# EUROPEAN PATENT APPLICATION

(11) **EP 4 410 286 A1**
(43) Date of publication of application: **07.08.2024**
(21) Application number: 22875168.1
(22) Date of filing: 30.09.2022
(51) Int. Cl.: A61K 31/4245, A61K 31/352, A61P 25/28, A61K 9/08, A61K 9/10, A61K 9/107, A61K 9/113, A61K 9/14, A61K 9/16, A61K 9/22, A61K 9/24, A61K 9/28, A61K 9/48

(54) **PHARMACEUTICAL COMPOSITION FOR PREVENTING OR TREATING ALZHEIMER'S DISEASE**

(30) Priority: 30.09.2021 CN 202111161273
(71) Applicant: Shandong New Time Pharmaceutical Co., Ltd., Linyi City, Shandong 273400 (CN)
(72) Inventor: ZHANG, Guimin, Linyi, Shandong 276006 (CN); YAO, Jingchun, Linyi, Shandong 276006 (CN); LI, Honghua, Linyi, Shandong 276006 (CN); SUN, Chenghong, Linyi, Shandong 276006 (CN); ZHAO, Tao, Linyi, Shandong 276006 (CN)
(74) Representative: Petty, Catrin Helen
(86) International application number: PCT/CN2022/123274
(87) International publication number: WO 2023/051787

(57) **Abstract**

A pharmaceutical composition for preventing or treating Alzheimer's disease and use thereof are disclosed and belong to the technical field of medicines. The pharmaceutical composition includes
(Z)-3-(N-(3-bromo-4-fluorophenyl)-N'-hydroxyformamido)-4-(2-guanidinoethyl)amino)-1,2, 5-oxadiazole and anhydroicaritin. A pharmacodynamic experiment proves that the pharmaceutical composition achieves a synergistic therapeutic effect on treating Alzheimer's disease.

## Description

### Technical Field

The present invention belongs to the technical field of medicines, and relates to a pharmaceutical composition for treating Alzheimer's disease, particularly a pharmaceutical composition including (Z)-3-(N-(3-bromo-4-fluorophenyl)-N'-hydroxyformamido)-4-(2-guanidinoethyl)amino)-1,2, 5-oxadiazole and anhydroicaritin.

### Background Art

Dementia is a syndrome that centers on acquired cognitive impairment and causes obvious decline in the ability of a patient to live, learn, work, and socially communicate. The cognitive impairment of a patient relates to the abilities of memory, learning, orientation, understanding, judgment, calculation, language, visual space function, problem analysis and solving, etc. The patient is often accompanied by mental, behavioral, and personality abnormalities at some stage of the course of the disease. Dementia is described as "neurocognitive impairment" in the Diagnostic and Statistical Manual of Mental Disorders, 5th edition (DSM-V) of the American Psychiatric Association. The diagnosis of dementia in the International Statistical Classification of Diseases and Related Health Problems 10th Revision (ICD-10) of the World Health Organization requires inquiry of medical history and neuropsychological examination to confirm the intellectual decline.

Dementia is mainly manifested by progressive memory decline and cognitive impairment, severely reduces the daily behavior and self-management ability of a patient, and causes heavy burden to the families of the patient. The incidence of dementia is high throughout the year. According to the WHO prediction, patients with dementia in 2050 will exceed 110 million. The pathogenesis of dementia is complex and a unified and clear understanding has not yet been formed. The pathogenic factors mainly comprise genetic factors, neurotransmitters, immune factors and the like, and are related to central cholinergic system dysfunction, synaptic plasticity dysfunction, oxidative stress, neuroinflammatory reaction, excitatory neurotoxicity and the like, but the exact pathogenesis is unclear.

Clinically, there are a large number of diseases causing dementia, and the classification methods thereof are different. The disease is classified into degenerative dementia and non-degenerative dementia according to whether the disease is degenerative or not. The former mainly includes Alzheimer's Disease (AD), dementia with Lewy body (DLB), Parkinson disease with dementia (PDD), frontotemporal lobar degeneration (FTLD) and the like; and the latter includes vascular dementia (V Alzheimer's disease), normal pressure hydrocephalus, and dementia caused by other diseases such as craniocerebral injury, infection, immunity, tumor, poisoning, metabolic diseases and the like.

Alzheimer's disease, also known as senile dementia, is a primary degenerative disease of the central nervous system, with progressive dysmnesia, general intellectual decline, personality changes, psychobehavioral abnormalities, and cognitive impairment as the main clinical manifestations. An acetylcholine transferase and acetylcholine of hippocampus and neocortex of a patient are significantly reduced, dysfunction of cortical cholinergic neuron transmitters is caused, and senile plaque, neurofibrillary tangle, granular cell degeneration, and β-amyloid peptide (Aβ) deposition are used as main pathologic changes. At present, the disease is not clear and widely considered to be a nervous system degenerative disease related to various factors such as heredity, environment and the like.

With the increasingly deep knowledge of Alzheimer's disease, the concepts of mild cognitive impairment (MCI) and vascular cognitive impairment (VCI) have been developed successively. The MCI is a cognitive impairment state between normal aging and Alzheimer's disease and a clinical prodromal stage of Alzheimer's disease. There is 10%-15% of the MCI developing into dementia each year on average, most of which are Alzheimer's disease. The MCI is increasingly concerned and has been used as an important research object for early diagnosis and intervention of Alzheimer's disease.

At present, most drugs for clinically treating Alzheimer's disease are a cholinesterase inhibitor and a N-methyl-D-aspartic acid receptor (NMDAR) blocker. However, these drugs can only relieve some symptoms and cannot relieve the pathological process of Alzheimer's disease. Although a therapy targeting Aβ and Tau shows an attractive development prospect in an animal experiment, the therapy often fails in a clinical trial and is difficult to be transformed. These drugs have side effects on the gastrointestinal tract and the central nervous system, are liable to cause drug dependence, and do not have obvious enough therapeutic effects Therefore, clarifying the pathogenesis of Alzheimer's disease and finding an effective treatment means to solve an unmet clinical need have great social significance and economic value, and have become a key subject of medical research at home and abroad.

In the Research on Molecular Mechanism of Icaritin in Preventing Inflammatory Reaction of Alzheimer's Disease, Zhu Menglin indicated that in an Alzheimer's disease inflammation model, icaritin can inhibit excessive activation of microglia induced by LPS and improve the learning and memory ability of mice.

The invention patent with the application number of 201310362101.9 discloses that icaritin can promote β-catenin to be translocated into nuclei, reduce phosphorylation of a Tau protein, protect neuron cell activity, and inhibit neurofibrillary tangle through a blood brain barrier and by inhibiting GSK-3 activity, thereby preventing and treating Alzheimer's disease.

### Summary

In order to find an effective therapeutic drug for Alzheimer's disease, the inventor creatively combines (Z)-3-(N-(3-bromo-4-fluorophenyl)-N'-hydroxyformamido)-4-(2-guanidinoethyl)amino)-1,2, 5-oxadiazole (compound I) and anhydroicaritin for treating Alzheimer's disease.

A pharmacodynamic experiment proves that the pharmaceutical composition of the present invention synergically relieves cognitive impairment of mice with Alzheimer's disease and rats with VD, increases the levels of 5-hydroxytryptamine and tryptophan in serum, reduces the production of L-kynurenine, and improves memory. On the basis of the above, the present invention provides a pharmaceutical composition whose active ingredients include anhydroicaritin and a compound I.

Further, when the weight ratio range of the compound I to the anhydroicaritin of the pharmaceutical composition of the present invention is (0.5-10):1, especially (1.6-3.3):1, the therapeutic effect of Alzheimer's disease is more excellent.

On the basis of the above, the present invention provides a pharmaceutical composition. The pharmaceutical composition comprises (Z)-3-(N-(3-bromo-4-fluorophenyl)-N'-hydroxyformamido)-4-(2-guanidinoethyl)amino)-1,2, 5-oxadiazole and anhydroicaritin.

In some embodiments of the present invention, the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (0.5-10): 1.

In some embodiments of the present invention, the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (1-5): 1.

In one embodiment of the present invention, the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (1.6-3.3): 1.

In some embodiments of the present invention, the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (2-3): 1.

In some embodiments of the present invention, the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is 1.6:1.

Pharmacodynamic example 1 shows a test of an effect of the pharmaceutical composition containing a compound I and anhydroicaritin of the present invention on mice in a model of Alzheimer's disease. The results show that the pharmaceutical composition containing a compound I and anhydroicaritin of the present invention obviously reduces the average exploration time, working memory error frequency, and reference memory error frequency, significantly relieves the cognitive impairment of mice, obviously increases the levels of 5-hydroxytryptamine and tryptophan in serum, and reduces the level of L-kynurenine in serum of the mice in a model of Alzheimer's disease. The pharmaceutical composition of the present invention effectively regulates the synthesis of monoamine neurotransmitters, inhibits the *in-vivo* metabolism of tryptophan, and improves the learning and memory ability.

The present invention provides use of the pharmaceutical composition containing a compound I and anhydroicaritin of the present invention in the preparation of a drug for preventing or treating Alzheimer's disease.

The present invention provides use of the pharmaceutical composition containing a compound I and anhydroicaritin of the present invention in the preparation of a drug for relieving cognitive impairment of a patient with Alzheimer's disease.

The pharmaceutical composition containing a compound I and anhydroicaritin of the present invention increases the levels of 5-hydroxytryptamine and tryptophan in serum, reduces the content of L-kynurenine in serum, and improves memory.

The present invention provides a pharmaceutical composition including (Z)-3-(N-(3-bromo-4-fluorophenyl)-N'-hydroxyformamido)-4-(2-guanidinoethyl)amino)-1,2, 5-oxadiazole (compound I) and anhydroicaritin. In view of the physicochemical properties of the composition of the present invention suitable for various routes of administration, these pharmaceutical compositions may be prepared according to well-known methods for preparing pharmaceutical compositions. The composition of the present invention may be formulated into any formulations suitable for human or animal by combining the composition of the present invention with one or more pharmaceutically acceptable solid or liquid excipients and/or adjuvants.

The pharmaceutical composition of the present invention may be administered in the form of unit dose. The administration route may be mainly digestive tract administration, such as oral administration, intestinal administration, etc. However, in view of the physicochemical properties of some compositions of the present invention suitable for parenteral administration, the forms of the parenteral administration are also acceptable, such as intravenous, intramuscular, subcutaneous and intraperitoneal injections, nasal, oromucosal, ocular, pulmonary and respiratory administrations, application to the skin and the like. In the use of treating Alzheimer's disease, the outstanding advantage is that the pharmaceutical composition may be prepared into a common oral formulation form such as a common tablet and a common capsule, a formulation that can be directly taken orally without special treatment (very convenient to use), and may also be prepared into other formulations including an injection via other various administration routes and modes.

The pharmaceutical composition of the present invention is prepared into a solid formulation, wherein a solid oral formulation is a tablet, a capsule, a granule, or a pill. The tablet comprises a common tablet, a coated tablet, a sugar-coated tablet, a film-coated tablet, an enteric-coated tablet, a multi-layered tablet, a dispersible tablet, a sustained-release tablet, etc. The pharmaceutical composition of the present invention has the advantages of convenient carrying, simple and convenient administration, and easy acceptance by a patient.

Other administration formulations may also be used in oral administration or other administration routes, including various liquid formulations, solid formulations, or semi-solid formulations prepared using novel techniques. The liquid formulation may be a solution (including a true solution and a colloidal solution), an emulsion (including O/W type, W/O type and multiple emulsions), a suspension, an injection (including a water injection, a powder injection, and an infusion solution), an eye drop, a nose drop, a lotion, a liniment, etc.; the solid formulation may be a tablet (including a common tablet, an enteric tablet, a buccal tablet, a dispersible tablet, a chewable tablet, an effervescent tablet, and an orally disintegrating tablet), a capsule (including a hard capsule, a soft capsule, and an enteric capsule), a granule, a powder, a pellet, a dropping pill, a suppository, a film agent, a patch, an aerosol (dry powder inhalation), a spray, etc.; and the semisolid formulation may be an ointment, an emulsifiable paste, a gel, a paste, etc.

The pharmaceutical composition of the present invention may be prepared into a common formulation, and also a sustained-release formulation, a controlled-release formulation, a targeting formulation, and various particle delivery systems.

In order to prepare the composition of the present application into a tablet, various excipients well-known in the related art, including a diluent, a binder, a wetting agent, a disintegrant, a lubricant, and a glidant, may be widely used. The diluent may be starch, dextrin, sucrose, glucose, lactose, mannitol, sorbitol, xylitol, microcrystalline cellulose, calcium sulfate, calcium hydrogen phosphate, calcium carbonate, etc.; the wetting agent may be water, ethanol, isopropanol, etc.; the binder may be starch slurry, dextrin, syrup, honey, a glucose solution, microcrystalline cellulose, acacia mucilage, gelatin slurry, sodium carboxymethylcellulose, methylcellulose, hydroxypropyl methylcellulose, ethyl cellulose, acrylic resin, carbomer, polyvinylpyrrolidone, polyethylene glycol, etc.; the disintegrant may be dry starch, microcrystalline cellulose, low-substituted hydroxypropyl cellulose, crosslinked polyvinylpyrrolidone, crosslinked sodium carboxymethylcellulose, sodium carboxymethyl starch, sodium bicarbonate, citric acid, polyoxyethylene sorbitan fatty acid ester, sodium dodecyl sulfate, etc.; and the lubricant and glidant may be talc powder, silicon dioxide, stearate, tartaric acid, liquid paraffin, polyethylene glycol, etc.

The tablet may be further prepared into a coated tablet, such as a sugar-coated tablet, a film-coated tablet, an enteric-coated tablet, or a double-layered tablet, and a multi-layered tablet.

In order to prepare an administration unit into a capsule, the pharmaceutical composition of the present invention may be mixed with a diluent and a glidant, and the mixture is directly placed into a hard capsule or a soft capsule; and the pharmaceutical composition of the present invention may also be prepared into a granule or a pellet with a diluent, a binder, and a disintegrant, and then the mixture is placed into a hard capsule or a soft capsule. The various diluents, binders, wetting agents, disintegrants, and glidants used for preparing the tablet of the pharmaceutical composition of the present invention may also be used for preparing a capsule of the pharmaceutical composition of the present invention.

In order to prepare the pharmaceutical composition of the present invention into an injection, water, ethanol, isopropanol, propylene glycol, or a mixture thereof may be used as a solvent, and a proper amount of a solubilizer, a cosolvent, a pH regulator, and an osmotic pressure regulator commonly used in the pharmaceutical field are be added. The solubilizer or cosolvent may be poloxamer, lecithin, hydroxypropyl-β-cyclodextrin, etc.; the pH regulator may be phosphate, acetate, hydrochloride, sodium hydroxide, etc.; and the osmotic pressure regulator may be sodium chloride, mannitol, glucose, phosphate, acetate, etc. For example, in the preparation of a freeze-dried powder injection, mannitol, glucose, etc. may further be added as a proppant.

Furthermore, if there are specific requirements on the formulation, a colorant, a preservative, a spice, a flavoring agent, or other additives may also be added into the pharmaceutical formulation.

For a pharmaceutical purpose and to enhance the therapeutic effect, the pharmaceutical composition of the present invention may be administered and used using any of the well-known administration and use methods.

The compound I of the present invention contains a pharmaceutically acceptable salt thereof.

The term "pharmaceutically acceptable salt" used herein refers to a salt that retains the biological efficacy of free acids and free bases of the specified compound and has no adverse effects biologically or in other aspects. The compound of the present application further comprises a pharmaceutically acceptable salt. The pharmaceutically acceptable salt refers to the form in which a base group in a parent compound is converted to a salt. The pharmaceutically acceptable salt includes, but is not limited to, an inorganic acid or organic acid salt of the base group such as an amino group. The pharmaceutically acceptable salt of the present application may be synthesized from the parent compound by reacting a basic group in the parent compound with 1-4 equivalents of an acid in a solvent system. A suitable salt is listed in one or more of Remingtong's Pharmaceutical sciences, 17th ed., Mack Publishing Company, Easton, Pa., 1985, p. 1418 and Journal of Pharmaceutical Science, 66, 2 (1977).

The term "treating" used herein and other similar synonyms include alleviating, reducing, or relieving a disease or a symptom of a disorder, inhibiting a disease or a disorder, e.g., arresting the development of a disease or a disorder, alleviating a disease or a disorder, alleviating a symptom caused by a disease or a disorder, or halting a symptom of a disease or disorder, preventing another symptom, and alleviating or preventing an underlying metabolic cause that causes a symptom. In addition, the term contains prophylactic purposes. The term further comprises obtaining a therapeutic effect and/or a prophylactic effect. The therapeutic effect refers to curing or relieving an underlying disease being treated. In addition, curing or relieving one or more physiological symptoms associated with an underlying disease is also a therapeutic effect, e.g., an improvement in a patient's condition is observed, although the patient may still be affected by the underlying disease. For the prophylactic effect, the composition may be administered to a patient at risk of developing a particular disease, or to a patient having one or more physiological symptoms of the disease, even if a diagnosis of the disease has not yet been made.

The term "effective amount", "therapeutically effective amount", or "pharmaceutically effective amount" used herein refers to the amount of at least one active substance (e.g., the compound of the present application) that is sufficient to alleviate one or more symptoms of the disease or disorder being treated to some extent after administration. The result may be a reduction and/or alleviation of signs, symptoms, or causes, or any other desired change in a biological system. For example, the "effective amount" for treatment is the amount of the composition containing the compound disclosed herein that is clinically necessary to provide an obvious effect for alleviating a disorder. The effective amount suitable in any individual case may be determined using techniques such as a dose escalation test.

Compared with the prior art, the pharmaceutical composition containing a compound I and anhydroicaritin provided by the present invention has the following technical advantages:

1. The pharmaceutical composition containing a compound I and anhydroicaritin of the present invention has synergistic technical effects on treating Alzheimer's disease and relieving cognitive impairment.

2. The pharmaceutical composition containing a compound I and anhydroicaritin of the present invention remarkably increases the levels of 5-hydroxytryptamine and tryptophan in serum, inhibits tryptophan metabolism, reduces the level of L-kynurenine, and improves memory.

3. The anhydroicaritin of the present invention is a natural traditional Chinese medicine monomer extracted from traditional Chinese medicine epimedium herb, has low toxic and side effects on human body, and may remarkably improve the medication safety and compliance of a patient, thereby greatly improving the therapeutic effect and life quality of the patient. In addition, the pharmaceutical composition containing a compound I and anhydroicaritin of the present invention may reduce the dosage, reduce the toxic and side effect of the drug, and has high safety while realizing the therapeutic effect of Alzheimer's disease.

### Detailed Description of Embodiments

The present invention will now be further illustrated by the following examples, but the scope of application of the present invention is not limited to the following examples, which are not intended to limit the present invention in any way, and any modifications, substitutions, etc. made by those skilled in the art within the technical essence of the present invention are within the scope of the technical solution of the present invention.

Epimedium flavonoid ingredients exist in the form of glycoside compounds in the plant epimedium herb. Anhydroicaritin has very low content in the original plant and is difficult to be directly separated and purified, such that the anhydroicaritin may be prepared by a method for hydrolyzing glycosidic bonds in epimedium flavonoid glycoside compounds. A method for preparing anhydroicaritin from icariin as a raw material comprises enzymolysis, acid hydrolysis, and a combination of enzymolysis and acid hydrolysis. Methods for hydrolyzing glycosidic bonds have been reported: (1) chemical methods, such as acid and alkaline hydrolysis. Hydrochloric acid, sulfuric acid, or nitric acid is mostly used in the acid hydrolysis, and sodium hydroxide, or potassium hydroxide is mostly used in the alkaline hydrolysis. The method mostly hydrolyzes a 3-rhamnose glycosidic bond and a 7-glucosyl glycosidic bond of icariin to obtain anhydroicaritin. The icariin is used as a reaction substrate in the research. (2) A bioconversion method comprises preparing a mold-containing solution by using epimedium herb as an inducer for fermentation and enzyme production of bacteria, mold, and yeast, and then converting flavonoid glycoside compounds of epimedium herb into low glycoside or aglycone. There are various flavonoid glycoside compounds, and a glycosidic bond structure thereof is very complicated and mainly composed of a glycosidic bond of α-L-rhamnose , a glycosidic bond of β-D-glucose, a glycosidic bond of β-D-xylose, and a glycosidic bond of other monosaccharides, and a glycosidic bond of a disaccharide composed thereof. The Chinese patent CN201711485889.7, entitled "Method for Preparing Anhydroicaritin", provides a method for preparing anhydroicaritin. On the basis of the various preparation methods, the anhydroicaritin may be prepared.

The anhydroicaritin in the present invention may be prepared from icariin as a raw material, may be prepared by chemical synthesis, or is commercially available.

### Example 1 Common tablet of pharmaceutical composition of present invention

Anhydroicaritin 3.2 g
Compound I 10.6 g
Starch 140 g
Dextrin 120 g
50% ethanol Appropriate amount
Magnesium stearate 1.0 g
Preparation process: anhydroicaritin, a compound I, starch, and dextrin were weighed according to the prescription amount, and uniformly mixed. An appropriate amount of 50% ethanol was added into the mixed powder to be uniformly mixed to prepare a soft material, the soft material was prepared into a wet granule through a 18-mesh nylon sieve, the wet granule was dried at about 60°C until the water content of the dry granule was controlled below 1.5%, the granule was separated through a 20-mesh sieve, and then mixed with magnesium stearate, and the mixture was tableted to obtain a finished product.

### Example 2 Capsule of pharmaceutical composition of present invention

Anhydroicaritin 10.6 g
Compound I 5.3 g
Microcrystalline cellulose 300 g
Superfine silica powder 12 g
Preparation process: anhydroicaritin, a compound I, microcrystalline cellulose, and superfine silica powder were crushed and sieved with a 100-mesh sieve, and uniformly mixed, and the mixed powder was directly filled into a capsule to obtain a finished product.

### Example 3 Dispersible tablet of pharmaceutical composition of present invention

Anhydroicaritin 1.6 g
Compound I 16 g
Calcium hydroxymethylcellulose 15 g
Microcrystalline cellulose 140 g
10% starch slurry Appropriate amount
Crosslinked polyvinylpyrrolidone 15 g
Magnesium stearate 6 g
Preparation process: anhydroicaritin and a compound I according to the prescription amount were sieved by a 100-mesh sieve, calcium hydroxymethylcellulose, crosslinked polyvinylpyrrolidone, and microcrystalline cellulose were sieved by a 80-mesh sieve, the materials were uniformly mixed, an appropriate amount of 10% starch slurry was added for granulation, magnesium stearate was added, and the mixture was tableted.

### Example 4 Granule of pharmaceutical composition of present invention

Anhydroicaritin 10 g
Compound I 16 g
Starch 200 g
Dextrin 50 g
Sucrose powder 50 g
80% ethanol Appropriate amount
Preparation process: anhydroicaritin, a compound I, starch, dextrin, and sucrose powder according to the prescription amount were weighed, and uniformly mixed. An appropriate amount of 80% ethanol was added into the mixed powder to be uniformly mixed to prepare a soft material, the soft material was prepared into a wet granule through a 18-mesh nylon sieve, the wet granule was dried at about 60°C, and the granule was separated through a 20-mesh sieve and sub-packaged to obtain a finished product.

### Example 5 Sustained-release tablet of pharmaceutical composition of present invention

Anhydroicaritin 21.2 g
Compound I 10.6 g
Hydroxypropylmethylcellulose 80 g
5% polyvinylpyrrolidone aqueous solution Appropriate amount
Lactose 85 g
Superfine silica powder 10 g
Preparation process: anhydroicaritin, a compound I, hydroxypropylmethylcellulose, and lactose according to the prescription amount were uniformly mixed, a 5% polyvinylpyrrolidone aqueous solution was added for granulation, the granule was dried at 40-80°C and separated, the prescription amount of superfine silica powder was added into the dry granule and uniformly mixed, and the mixture was stamped in a special shape.

### Example 6 Dropping pill of pharmaceutical composition of present invention

Anhydroicaritin 10 g
Compound I 16 g
Polyethylene glycol-6000 72.5 g
Polyethylene glycol-1000 25.0 g
Preparation process: anhydroicaritin and a compound I according to the prescription amount were sieved through a 100-mesh sieve, and added into a mixed solution heated and melted on a water bath and containing polyethylene glycol-6000 and polyethylene glycol-1000 according to the prescription, the materials were fully stirred to be uniform, and the mixture was filled into a dropping bottle to be dropped at 95±2°C, that is, the mixture was dropped into a glass condensation column containing 4-6 mL of methyl silicone oil, molded, and taken out, the adhered methyl silicone oil was removed by absorbing with absorbent paper, and a finished product was obtained.

### Example 7 Enteric-coated soft capsule of pharmaceutical composition of present invention

Prescription of contents:
Anhydroicaritin 1.6 g
Compound I 16 g
Anhydrous ethanol 10 g
Polyoxyethylene castor oil 50 g
Medium-chain fatty acid glycerides 20 g
Prescription of gel skin:
Gelatin 10 g
Glycerin 5 g
Purified water 10 g
Prescription of enteric coating solution:
Eudragit L30D-55 100 g
Triethyl citrate 10 g
Talc powder 7.5 g
Purified water 200 g
Preparation process: medium-chain fatty acid glycerides, polyoxyethylene castor oil, 1,2-propylene glycol, and absolute ethyl alcohol were weighed according to the prescription amount, uniformly mixed, and stirred, and the mixture was dissolved with a compound I and anhydroicaritin, or ultrasonic treatment may also be performed to accelerate dissolution to obtain a clear concentrated solution, namely a microemulsion concentrate of a compound I and anhydroicaritin. The obtained microemulsion concentrate was diluted with water according to the weight ratio of 1:10-20 to a clear solution, that is, the microemulsion content of a soft capsule. Gelatin, glycerin, and purified water according to the prescription amount were weighed, uniformly mixed, and pressed into a gel skin, and then Eudragit L30D-55, triethyl citrate, talc powder, and purified water according to the prescription amount were weighed, and uniformly mixed to obtain an enteric coating solution. The microemulsion content of a soft capsule containing a compound I and anhydroicaritin was wrapped by the gel skin to prepare a soft capsule, and a casing was wrapped on the soft capsule to prepare an enteric-coated soft capsule.

### Example 8 Common tablet of pharmaceutical composition of present invention

Anhydroicaritin 10 g
Compound I 16 g
Starch 260 g
Dextrin 220 g
50% ethanol Appropriate amount
Magnesium stearate 1.8 g
Preparation process: anhydroicaritin, a compound I, starch, and dextrin were weighed according to the prescription amount, and uniformly mixed. An appropriate amount of 50% ethanol was added into the mixed powder to be uniformly mixed to prepare a soft material, the soft material was prepared into a wet granule through a 18-mesh nylon sieve, the wet granule was dried at about 60°C until the water content of the dry granule was controlled below 1.5%, the granule was separated through a 20-mesh sieve, and then mixed with magnesium stearate, and the mixture was tableted to obtain a finished product.

### Example 9 Capsule of pharmaceutical composition of present invention

Anhydroicaritin 10 g
Compound I 16 g
Microcrystalline cellulose 500 g
Superfine silica powder 20 g
Preparation process: anhydroicaritin, a compound I, microcrystalline cellulose, and superfine silica powder were crushed and sieved with a 100-mesh sieve, and uniformly mixed, and the mixed powder was directly filled into a capsule to obtain a finished product.

### Example 10 Granule of pharmaceutical composition of present invention

Anhydroicaritin 10.6 g
Compound I 5.3 g
Starch 120 g
Dextrin 30 g
Sucrose powder 300 g
80% ethanol Appropriate amount
Preparation process: anhydroicaritin, a compound I, starch, dextrin, and sucrose powder according to the prescription amount were weighed, and uniformly mixed. An appropriate amount of 80% ethanol was added into the mixed powder to be uniformly mixed to prepare a soft material, the soft material was prepared into a wet granule through a 18-mesh nylon sieve, the wet granule was dried at about 60°C, and the granule was separated through a 20-mesh sieve and sub-packaged to obtain a finished product.

In order to verify the efficacy of the pharmaceutical composition containing a compound I and anhydroicaritin in treating Alzheimer's disease, the inventor carries out related a pharmacodynamic test. It should be noted that drugs selected in the pharmacodynamic test described below are obtained by the representative formula of the present invention and a preparation method therefor. The inventor also carries out a pharmacodynamic experiment on drugs obtained by other formulas contained in the present invention and preparation methods therefor. The experimental results show that the drugs obtained by other formulas and preparation methods thereof have the same or similar effects, but the drugs are not exhaustive due to space limitations. In addition, the pharmacodynamic experiment described below demonstrates the efficacy of the present invention only takes a partially representative animal model as an example.

### Pharmacodynamic example 1 Effect of pharmaceutical composition containing compound I and anhydroicaritin of present invention on mice in model of Alzheimer's disease

An aluminum element has been reported to induce pathological changes in an animal brain, but the dosage and time are inconsistent, the dosage is 10.8-500 mg/kg⁻¹, and the time is from 6 weeks to 3 months. After an animal is injected with D-galactose for a long time, since the latter metabolite galactitol cannot be further metabolized and is accumulated in cells, an osmotic pressure is influenced, cell swelling and metabolic disturbance are caused, active oxygen *in vivo* is increased, lipids in a cell membrane are damaged, functions of multiple organs and multiple systems of the body are degraded, and the phenomena are very similar to the aging changes. D-galactose further causes a range of degenerative changes in brain neurons, including a reduction in the number of neurons, a decrease in the activity of superoxide dismutase in a brain tissue, and elevated levels of malondialdehyde and lipofuscin.

A food providing device is arranged at an end of each arm of an eight-arm maze. The spatial memory ability of an experimental animal may be reflected according to the strategy of analyzing feeding of an animal, namely the parameters of the times, time, correct times, wrong times, routes and the like of entering each arm. In contrast, the eight-arm maze is simple, convenient and feasible to operate, can distinguish short-term working memory from long-term reference memory, and is widely used for evaluating a learning and memory cognitive experiment. The main analysis indexes of the eight-arm maze are name, grouping, working memory error latency, reference memory error latency, total correct times, total arm entering times, working memory error times, reference memory error times, correct latency, correct rate, working memory error rate, reference memory error rate, correct times before first error, test time, a track graph and the like.

There are two main factors affecting the operation of the animal maze: fear of the maze or an observer and driving by animal exploration habits and food known to be placed in the arms of the maze. The fear factor is too strong to prevent the maze operation of the animal, such that the animal stays in a certain place of the maze all the time without exploring. Lack of craving for food also produces a similar result. Increasing strokes of the animal and heightening side walls of the maze arms if necessary help to reduce the animal's fear. If the driving effect of food is insufficient, the amount of food can be reduced, but the weight and general physical condition must be monitored simultaneously.

The eight-arm maze is used to perform a learning and memory test, indicating that D-galactose and AlCl₃ can cause the learning and memory ability of mice to be reduced, and a model of Alzheimer's disease is successfully modeled.

### 1. Experimental animal

100 SPF-grade Kunming mice, each half male and female, weighed 20-22 g, were provided by the Jinan Pengyue Experimental Animal Breeding Co., Ltd with the animal certificate number: SCXK(Lu)20190003. The mice were bred to freely drink and eat in an SPF-grade animal breeding room with strictly controlled environmental conditions: the temperature of 20-26°C, the humidity of 40-70%, the light and dark cycle of 12 h: 12 h, and a growing and breeding feed.

### 2. Main instruments and reagents

Compound I provided by Shandong New Time Pharm Co., Ltd;
anhydroicaritin provided by Shandong New Time Pharm Co., Ltd;
eight-arm maze; and
high performance liquid chromatography.

### 3. Experimental method and experimental grouping

All mice were first trained in an eight-arm maze which was a common model for evaluating the learning and memory ability of an animal. The animals were adaptively fed for 1 week, weighed, and fasted for 24 hours. Then after each day of training, the mice were restrictively given a normal diet to maintain the body weight of the mice between 80% and 85% of that of mice fed normally. On the second day, food particles (4-5 particles each arm with the diameter of about 3-4 mm) were sprayed in each arm and a center area of the maze. Then 4 animals were placed in the center of the maze simultaneously (doors to each arm were opened). The animals were freely fed and explored for 10 min. On the third day, the training of the second day was repeated. This process allowed the animals to become familiar with the maze environment without very strong stress. On the fourth day, the animals were trained individually: one food particle was placed in each arm near an outer food box and the animals ate freely. The animals were removed after the food particles were consumed or 10 min later. On the fifth day, the food was placed in the food box and the previous day of training was repeated 2 times a day. After the sixth day, 4 arms were randomly selected and one food particle was placed in each arm; each arm door was closed and the animals were placed in the center of the maze; and after 30s, the arm door was opened, the animals moved freely in the maze and took the food particle until the animals ate the food particles of all 4 arms. If the food particles were not eaten up after 10 min, the experiment was terminated. The animals were trained twice a day with the interval of 1 h or more. When the working memory error of 5 continuous trainings was zero and the reference memory error did not exceed 1 time, the molding was started.

The mice meeting the learning standard were screened, 10 of them were randomly selected as a normal group, 10 as a model group, and the others as experimental groups. Except that the mice in the normal group were intraperitoneally injected with the same amount of normal saline and intragastrically administrated with the same amount of double distilled water, the mice of the other groups were all intraperitoneally injected with D-galactose (500 mg/kg/D) (prepared with normal saline) and intragastrically administrated AlCl₃ (20 mg/kg/D) (prepared with double distilled water).

The mice in the experimental groups were intragastrically administrated once a day according to the grouping and administration dose and the mice in the normal group and the model group were intragastrically administrated with the same amount of purified water for 8 weeks successively. The mice in the experimental groups were divided into 8 groups of low- and high-dose anhydroicaritin groups, low- and high-dose compound I groups, and composition A, B, C, and D groups with 10 mice in each group. Grouping and administration dose of each group were as follows:
Low-dose anhydroicaritin group: intragastrically administrated with 3.2 mg/(kg.d) of anhydroicaritin
High-dose anhydroicaritin group: intragastrically administrated with 21.2 mg/(kg.d) of anhydroicaritin
Low-dose compound I group: intragastrically administrated with 0.6 mg/(kg.d) of a compound I
High-dose compound group: intragastrically administrated with 32 mg/(kg.d) of a compound I
Composition A group: intragastrically administrated with 5.3 mg/(kg.d) of a compound I+1.6 mg/(kg.d) of anhydroicaritin
Composition B group: intragastrically administrated with 5.3 mg/(kg.d) of a compound I+10.6 mg/(kg.d) of anhydroicaritin
Composition C group: intragastrically administrated with 16 mg/(kg.d) of a compound I+1.6 mg/(kg.d) of anhydroicaritin
Composition D group: intragastrically administrated with 16 mg/(kg.d) of a compound I+10 mg/(kg.d) of anhydroicaritin
A statistical method was analyzed by an SPSS19.0 statistical software, the measurement data was expressed as *x̅*±s, t test and variance analysis were used, and a *P* < 0.05 indicated that the difference was statistically significant.

### 3.1 Behavioral experiment:

The mice in each group were respectively placed in the center of the eight-arm maze for an experiment, and the working memory error, reference memory error, total arm entering times, and test time were recorded.

The working memory error frequency, reference memory error frequency, and average exploration time were calculated according to the recorded working memory error, reference memory error, total arm entering times, and test time:
working memory error frequency = working memory error times/total arm entering times;
reference memory error frequency = reference memory error times/total arm entering times; and
average exploration time = test time/total arm entering time.

### 3.2 Preparation of serum

After the last administration, 2 ml of blood was taken from each eyeball of each group of the mice subjected to the eight-arm maze test and stood overnight at 4°C in a test tube, and a supernatant was taken the following day for later use.

The contents of tryptophan, L-kynurenine, and 5-hydroxytryptamine in blood serum were detected by a high performance liquid chromatography.

### 4. Experimental results

### 4.1. Behavioral experimental results

**Table 1 Comparison of average exploration time, working memory error frequency, and reference memory error frequency of mice in each group (x̅ ± s, n = 10)**

| Groups | Average exploration time (s) | Working memory error frequency | Reference memory error frequency |
|---|---|---|---|
| Model group | 191.75±0.75 | 0.52±0.03 | 0.23±0.04 |
| Low-dose compound I group | 190.20±1.09⁺ | 0.52±0.03⁺⁺ | 0.23±0.05⁺ |
| High-dose compound I group | 189.95±1.04⁺ | 0.44±0.05⁺ | 0.21±0.04⁺ |
| Low-dose anhydroicaritin group | 190.17±1.18⁺ | 0.45±0.02⁺ | 0.21±0.03⁺ |
| High-dose anhydroicaritin group | 189.81±1.19⁺ | 0.44±0.06⁺ | 0.20±0.04⁺ |
| Composition A group | 186.52±0.065^{+++ ★ ★ ★ &&&**###◊∘} | 0.36±0.07^{+++★&**#} | 0.14±0.04^{+++★&&**##} |
| Composition B group | 188.10±1.39^{+++★&*#} | 0.37±0.05^{+++★&**#} | 0.15±0.04^{+++★&*#} |
| Composition C group | 188.19±1.37^{+++★&*#} | 0.35±0.05^{+++★&&**##} | 0.14±0.03^{+++ ★ ★ &&**##} |
| Composition D group | 186.42±0.62^{+++ ★ ★ ★ &&***###△◊◊∘∘} | 0.32±0.08^{+++★&&*#△} | 0.11±0.05^{+++★★&&*#△} |

| | | | |
|---|---|---|---|
| Note: compared with the model group, ⁺*P* < 0.05, ⁺⁺*P* < 0.01, and ⁺⁺⁺*P* < 0.001; compared with the low-dose compound I group, *^{&}P* < 0.05, *^{&&}P* < 0.01, and *^{&&&}P* < 0.001; compared with the high-dose compound I group, ^{★}*P* < 0.05, ^{★★}*P* < 0.01, and ^{★★★}*P* < 0.001; compared with the low-dose anhydroicaritin group, **P* < 0.05, ***P* < 0.01, and ****P* < 0.001; compared with the high-dose anhydroicaritin group, *^{#}P* < 0.05, *^{# #}P <* 0.01, and ^{# # #}*P* < 0.001; compared with composition A group, ^{△}*P* < 0.05, ^{△△}*P* < 0.01, and ^{△△△}*P* < 0.001; compared with composition B group, ^{◊}*P* < 0.05, ^{◊◊}*P* < 0.01, and ^{◊◊◊}*P* < 0.001; and compared with composition C group, ^{∘}*P* < 0.05, ^{∘∘}*P* < 0.01, and *^{∘∘∘}P* < 0.001. | | | |

The experimental results showed that the pharmaceutical composition containing a compound I and anhydroicaritin obviously reduced the average exploration time, working memory error frequency, and reference memory error frequency of the mice in a model of Alzheimer's disease, obviously improved cognitive impairment of the mice, and had a better effect than the high- and low-dose compound I groups and the high- and low-dose anhydroicaritin groups.

The effect of relieving cognitive impairment of mice was better in the composition D group and suboptimal in the composition A group compared to that of the composition B and C groups.

### 4.2 Contents of tryptophan, L-kynurenine, and 5-hydroxytryptamine in serum

**Table 2 Comparisons of results of 5-hydroxytryptamine, tryptophan, and L-kynurenine in serum of mice in each group (x̅ ± s, n = 10)**

| Groups | 5-hydroxytryptamine (µg/ml) | Tryptophan (µg/ml) | L-kynurenine (µg/ml) |
|---|---|---|---|
| Model group Low-dose | 1.23±0.06 | 16.84±1.07 | 0.23±0.09 |
| compound I group | 1.29±0.05⁺ | 17.92±0.95⁺ | 0.35±0.03⁺ |
| High-dose compound I group | 1.28±0.05⁺ | 18.08±0.02⁺ | 0.33±0.01⁺ |
| Low-dose anhydroicaritin group | 1.29±0.05⁺ | 17.79±0.65⁺ | 0.33±0.01⁺ |
| High-dose anhydroicaritin group | 1.30±0.07⁺ | 17.90±0.30⁺ | 0.33±0.02⁺ |
| Composition A group | 1.39±0.02^{+++ ★ ★ ★ &&&***#◊∘} | 19.03±0.16^{+++ ★ ★ &&***###◊∘∘} | 0.30±0.01^{++ ★ ★ ★ &&&***#*◊∘∘∘} |
| Composition B group | 1.36±0.04^{+++★★&&**#} | 18.79±0.29^{+++★&***###} | 0.31±0.01^{++★★★&&&**#} |
| Composition C group | 1.36±0.04^{+++★&&**#} | 18.80±0.27^{+++★&***###} | 0.31±0.01^{+++★★&&&**#} |
| Composition D group | 1.39±0.01^{+++ ★ ★ ★ &&&***###Δ◊◊∘∘} | 19.29±0.31^{+++ ★ ★ &&***###△◊◊∘∘} | 0.29±0.01^{+++ ★ ★ ★ &&&***##△◊◊◊∘∘∘} |

| | | | |
|---|---|---|---|
| Note: compared with the model group, ⁺*P* < 0.05, ⁺⁺*P* < 0.01, and ⁺⁺⁺*P* < 0.001; compared with the low-dose compound I group, ^{&}*P* < 0.05, ^{&&}*P* < 0.01, and ^{&&&}*P* < 0.001; compared with the high-dose compound I group, ^{★}*P* < 0.05, ^{★★}*P* < 0.01, and ^{★★★}*P* < 0.001; compared with the low-dose anhydroicaritin group, *^{*}P* < 0.05, *^{**}P* < 0.01, and ^{***}*P* < 0.001; compared with the high-dose anhydroicaritin group, *^{#}P* < 0.05, *^{# #}P* < 0.01, and^{# # #} *P* < 0.001; compared with composition A group, ^{△}*P* < 0.05, ^{△△}*P* < 0.01, and ^{△△△}*P* < 0.001; compared with composition B group, ^{△}*P* < 0.05, ^{◊◊}*P* < 0.01, and ^{△△△}*P* < 0.001; and compared with composition C group, ^{°}*P* < 0.05, *^{∘∘}P* < 0.01, and *^{∘∘∘}P* < 0.001. | | | |

The experimental results showed that the pharmaceutical composition containing a compound I and anhydroicaritin obviously improved the levels of 5-hydroxytryptamine and tryptophan in serum of the mice in a model of Alzheimer's disease, obviously reduced the level of L-kynurenine in serum of the mice in a model of Alzheimer's disease, and had the effect better than the high- and low-dose compound I groups and high- and low-dose anhydroicaritin groups.

Compared with the compositions B and C, the composition D had better effects of increasing the contents of 5-hydroxytryptamine and tryptophan in serum and reducing the content of L-kynurenine, and the composition A had a suboptimal effect. The composition containing a compound I and anhydroicaritin of the present invention effectively regulated the synthesis of monoamine neurotransmitters, inhibited the *in-vivo* metabolism of tryptophan, and improved the learning and memory ability.

The pharmaceutical composition containing a compound I and anhydroicaritin of the present invention had a remarkable therapeutic effect on the model of Alzheimer's disease, obviously relieved cognitive impairment of the model of Alzheimer's disease, and enhanced memory. The two active ingredients of the compound I and the anhydroicaritin were synergistic, and had the remarkable therapeutic effect on Alzheimer's disease.

The above described examples are not intended to limit the present invention in any form. Any simple modifications, substitutions, etc. on the basis of the technical essence of the present invention without departing from the technical solution of the present invention should fall within the scope of the technical solution of the present invention.

## Claims

1. A pharmaceutical composition comprising (Z)-3-(N-(3-bromo-4-fluorophenyl)-N'-hydroxyformamido)-4-(2-guanidinoethyl)amino)-1,2, 5-oxadiazole and anhydroicaritin.

2. The pharmaceutical composition according to claim 1, wherein the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (0.5-10): 1.

3. The pharmaceutical composition according to claim 2, wherein the weight ratio of the (Z)-3-(N-(3-bromo-4-fluorophenyl)-N'-hydroxyformamido)-4-(2-guanidinoethyl)amino)-1,2, 5-oxadiazole to the anhydroicaritin is (1.6-3.3): 1.

4. The pharmaceutical composition according to any one of claims 1-3, wherein the pharmaceutical composition is prepared into a liquid formulation, a solid formulation, or a semi-solid formulation.

5. The pharmaceutical composition according to claim 4, wherein the solid formulation is selected from a tablet, a capsule, a granule, a powder, a pellet, or a dropping pill.

6. The pharmaceutical composition according to claim 4, wherein the liquid formulation is selected from a solution, an emulsion, a suspension, or an injection.

7. Use of the pharmaceutical composition according to any one of claims 1-6 in the preparation of a drug for preventing or treating Alzheimer's disease.

8. The use according to claim 7, wherein the pharmaceutical composition is used in the preparation of a drug for relieving cognitive impairment of a patient with Alzheimer's disease.

9. The use according to claim 7, wherein the pharmaceutical composition increases the levels of 5-hydroxytryptamine and tryptophan in serum, reduces the content of L-kynurenine in serum, and improves memory.

10. The use according to claim 7, wherein the weight ratio of (Z)-3-(N-(3-bromo-4-fluorophenyl)-N'-hydroxyformamido)-4-(2-guanidinoethyl)amino)-1,2, 5-oxadiazole to anhydroicaritin is (1.6-3.3): 1.
